# EUROPEAN PATENT APPLICATION

(11) **EP 4 656 349 A1**
(43) Date of publication of application: **03.12.2025**
(21) Application number: 23930899.2
(22) Date of filing: 11.12.2023
(51) Int. Cl.: B29C 33/42, A61B 1/00, B29C 33/00

(54) **MOLDING MOLD FOR ENDOSCOPE HOOD AND METHOD FOR MANUFACTURING ENDOSCOPE HOOD**

(30) Priority: 28.03.2023 JP 2023051723
(71) Applicant: Seed Co., Ltd., Tokyo 113-8402 (JP)
(72) Inventor: KANDA Kana, Tokyo 113-8402 (JP); SATO Takao, Tokyo 113-8402 (JP)
(74) Representative: Viering, Jentschura & Partner mbB Patent- und Rechtsanwälte
(86) International application number: PCT/JP2023/044298
(87) International publication number: WO 2024/202278

(57) **Abstract**

The purpose is to facilitate releasing of the endoscope hood after a copolymerization reaction and avoid liquid leakage during the copolymerization reaction. In a molding die for an endoscope hood, a hollow cylindrical member 20A is open at bottom surfaces, the hollow cylindrical member 20A is configured such that it is possible to inset the hollow cylindrical member 20A into a cavity 13 of a first member 10, an outer diameter r_{B} of an annular portion 30B is larger than an outer diameter r_{A} of a main body portion 30A, the main body portion 30A is configured such that it is possible to insert the main body portion 30A into the hollow cylindrical member 20A, a radius r₄ of a cavity 43 of a fourth member 40 is larger than an outer shape r₁₀ of a first member 10, and the first member 10 and the fourth member 40 are engageable with each other.

## Description

### TECHNICAL FIELD

The present invention relates to a molding die for an endoscope hood and a method for manufacturing the endoscope hood.

### BACKGROUND ART

During surgery or examination using an endoscope, a transparent hood is attached to an extension portion (insertion-side end portion) of the endoscope for the purpose of confirming an optimal distance from the extension portion of the endoscope to a biological tissue and of ensuring a field of view. The hood is made of ABS, polycarbonate, vinyl chloride, silicone rubber, or the like.

However, such a hood is attached for the purpose of confirming an optimal distance from the extension portion of the endoscope to the biological tissue and of ensuring a field of view but a camera lens and an illumination lens are not covered by the hood. Thus, there is a problem that body fluid or oil may adhere and the field of view may become unclear during the examination or surgery using an endoscope to which the hood is attached.

### CITATION LIST

### PATENT LITERATURE

Patent Literature 1: Japanese Patent Application Laid-Open No. H11-047081

### SUMMARY OF THE INVENTION

### TECHNICAL PROBLEM

In order to solve such a problem, it is conceivable to form an endoscope hood from a hydrogel formed using a hydrophilic monomer. As a method for forming the endoscope hood from a hydrogel, a material containing the hydrophilic monomer is filled into a molding die made of, for example, plastic, and is subjected to a copolymerization reaction by raising a temperature. The material subjected to the copolymerization reaction is hydrated and swollen to obtain an endoscope hood made of a hydrogel. A shape of the endoscope hood obtained by the method corresponds to a shape of the molding die filled with the material containing the hydrophilic monomer.

However, when the conventional molding die is used, there is a problem that adhesion of the endoscope hood to the molding die is high, and it is difficult to release the endoscope hood after the copolymerization reaction.

In addition, in the conventional molding die, when the material described above is injected into the molding die and subjected to the copolymerization reaction, sealing property is low, and liquid leakage is a problem.

Therefore, the present invention has been made to solve the problems described above, and an object of the present invention is to provide a molding die for an endoscope hood and a method for manufacturing the endoscope hood that can facilitate releasing of the endoscope hood after a copolymerization reaction and avoid liquid leakage during the copolymerization reaction.

### SOLUTION TO PROBLEM

A molding die for an endoscope hood according to an embodiment is summarized as including: a cylindrical first member that has a first bottom surface, a second bottom surface, and a cylindrical cavity opened in the second bottom surface; two semi-cylindrical second members that are coupled to each other to form a hollow cylindrical member; a third member that includes a cylindrical main body portion that has a first bottom surface, a second bottom surface, and a cylindrical cavity opened in the second bottom surface, and an annular portion provided on the second bottom surface; and a cylindrical fourth member that has a first bottom surface, a second bottom surface, and a cylindrical cavity opened in the second bottom surface, wherein the hollow cylindrical member is open at both bottom surfaces, the hollow cylindrical member is configured such that it is possible to inset the hollow cylindrical member into the cavity of the first member, an outer diameter of the annular portion is larger than an outer diameter of the main body portion, the main body portion is configured such that it is possible to insert the main body portion into the hollow cylindrical member, a radius of the cavity of the fourth member is larger than an outer shape of the first member, and the first member and the fourth member are engageable with each other.

A method for manufacturing an endoscope hood according to an embodiment, in which a cylindrical first member has a first bottom surface, a second bottom surface, and a cylindrical cavity opened in the second bottom surface, a third member includes a cylindrical main body portion that has a first bottom surface, a second bottom surface, and a cylindrical cavity opened in the second bottom surface, and an annular portion that is provided on the second bottom surface and has an outer radius larger than an outer shape of the main body portion, and a cylindrical fourth member has a first bottom surface, a second bottom surface, and a cylindrical cavity opened in the second bottom surface, the method is summarized as including steps of: coupling two semi-cylindrical second members to form a hollow cylindrical member opened at both bottom surfaces; inserting the hollow cylindrical member into the cavity of the first member; injecting a mixture of monomers containing a hydrophilic monomer into an opening of the hollow cylindrical member; inserting the main body portion of the third member into the opening of the hollow cylindrical member; and engaging an opening of the fourth member with an outer periphery of the first member to perform heat treatment.

### ADVANTAGEOUS EFFECTS OF INVENTION

According to the present invention, it is possible to provide a molding die for an endoscope hood and a method for manufacturing the endoscope hood that can facilitate releasing of the endoscope hood after a copolymerization reaction and avoid liquid leakage during the copolymerization reaction.

### BRIEF DESCRIPTION OF THE DRAWINGS

Fig. 1 is an example of a perspective view of a molding die for an endoscope hood according to an embodiment.
Fig. 2 is an example of a cross-sectional view taken along a line X-X of Fig. 1.
Fig. 3 is a view illustrating an example of a first member 10 of a molding die 1 for an endoscope hood according to the embodiment.
Fig. 4 is a view illustrating an example of a second member 20 of the molding die 1 for an endoscope hood according to the embodiment.
Fig. 5 is a view illustrating an example of a third member 30 of the molding die 1 for an endoscope hood according to the embodiment.
Fig. 6 is a view illustrating an example of a fourth member 40 of the molding die 1 for an endoscope hood according to the embodiment.
Fig. 7 is a view for describing an example of a method for manufacturing the endoscope hood according to the embodiment.

### DESCRIPTION OF EMBODIMENTS

Hereinafter, an embodiment will be described in detail with reference to the drawings as appropriate. However, unnecessarily detailed description may be omitted. For example, a detailed description of a well-known matter and a repeated description of substantially the same configuration may be omitted. This is to avoid unnecessary redundancy of the following description and to facilitate better understanding of those skilled in the art. Note that the inventors provide the accompanying drawings and the following description in order for those skilled in the art to fully understand the present disclosure, and do not intend to limit the subject matter described in the claims to the accompanying drawings and the following description.

In the following description of the drawings, the same or similar parts are denoted by the same or similar reference numerals. It should be noted that the drawings are schematic, and ratios of dimensions and the like may be different from actual ones. Therefore, specific dimensions and the like should be determined in consideration of the following description. In addition, the drawings may include parts having different dimensional relationships and ratios.

### <First Embodiment>

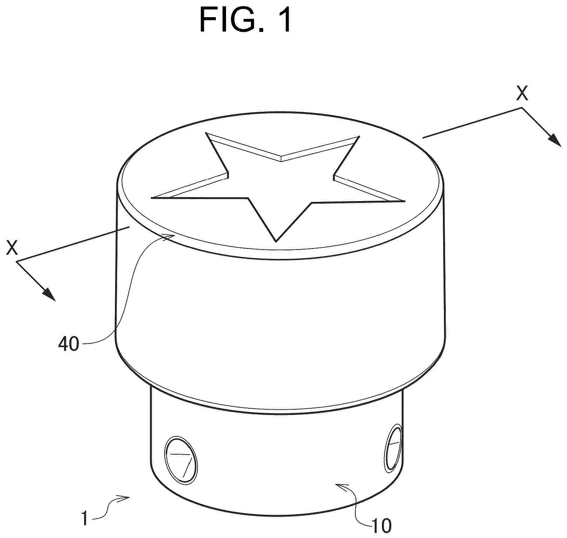

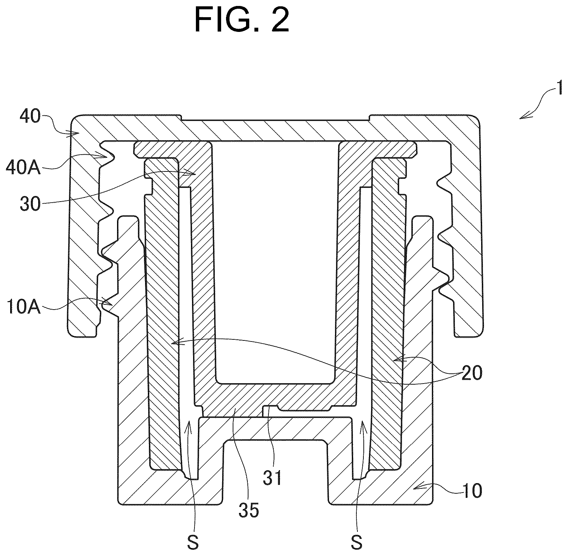

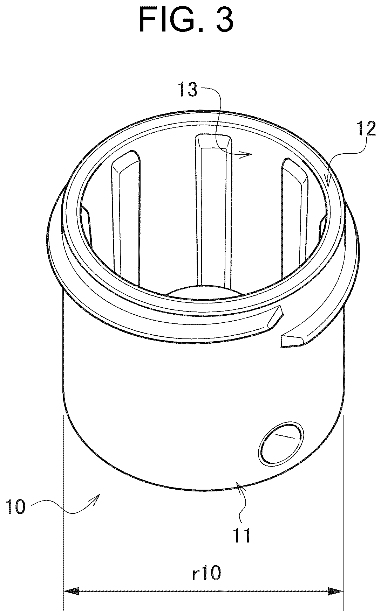

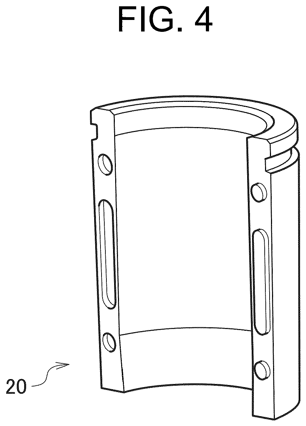

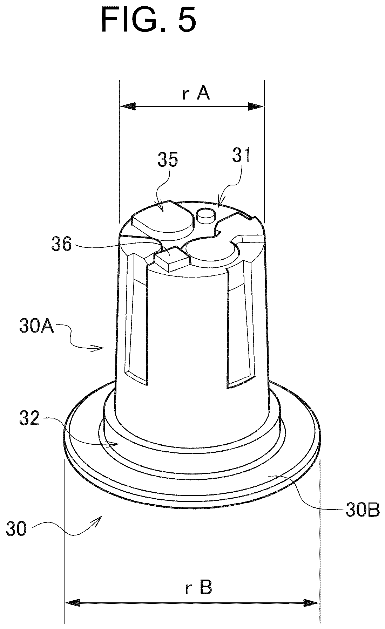

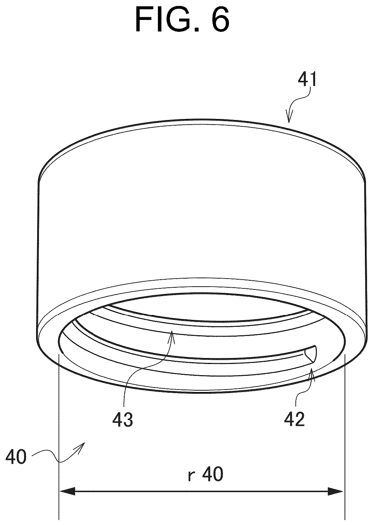

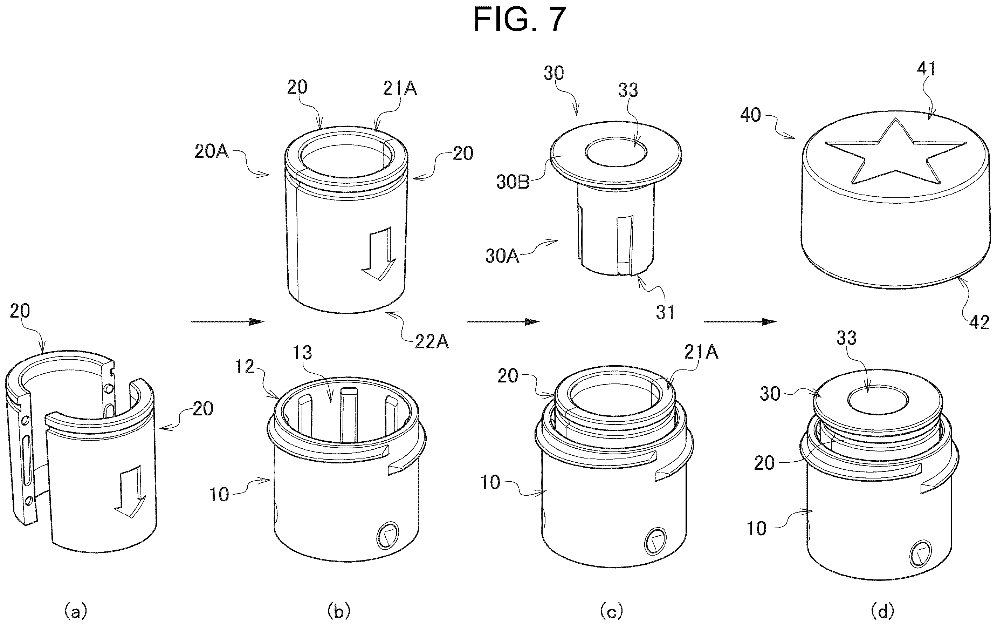

A molding die for an endoscope hood according to a first embodiment of the present invention will be described with reference to Figs. 1 to 7. Fig. 1 is a view illustrating an example of a perspective view of a molding die 1 for an endoscope hood according to the present embodiment, Fig. 2 is an example of a cross-sectional view taken along a line X-X of Fig. 1, Fig. 3 is a view illustrating an example of a first member 10 of the molding die 1 for an endoscope hood according to the present embodiment, Fig. 4 is a view illustrating an example of a second member 20 of the molding die 1 for an endoscope hood according to the present embodiment, Fig. 5 is a view illustrating an example of a third member 30 of the molding die 1 for an endoscope hood according to the present embodiment, Fig. 6 is a view illustrating an example of a fourth member 40 of the molding die 1 for an endoscope hood according to the present embodiment, and Fig. 7 is a view for describing an example of a method for manufacturing the endoscope hood according to the embodiment.

The molding die 1 for an endoscope hood according to the present embodiment is used for manufacturing an endoscope hood made of a hydrogel.

As illustrated in Figs. 1 and 2, the molding die 1 includes the first member 10, the second members 20, the third member 30, and the fourth member 40.

A dimension of each member can be selected as appropriate according to a shape of the target endoscope hood, and is not particularly limited.

In addition, synthetic resin used in the molding die 1 for an endoscope hood according to the present embodiment preferably has chemical resistance and heat resistance to an extent that it can withstand a step of injecting a mixture of monomers containing a hydrophilic monomer and subjecting the mixture to a thermal polymerization reaction, for example. The mixture of the monomers containing the hydrophilic monomer includes the hydrophilic monomer and a monomer copolymerizable therewith.

Examples of the synthetic resin include polyethylene, polypropylene, alicyclic polyolefin, polyethylene terephthalate, polybutylene terephthalate, polycarbonate, and thermoplastic elastomer. In the present invention, polypropylene is preferably used from a viewpoint of moldability.

As illustrated in Fig. 3, the first member 10 is a cylindrical member having a first bottom surface 11, a second bottom surface 12, and a cylindrical cavity 13 opened in the second bottom surface 12.

As illustrated in Fig. 4, the second members 20 are two semi-cylindrical members that are coupled to each other to form a hollow cylindrical member 20A. As illustrated in Figs. 2 and 7(b), the hollow cylindrical member 20A is open at both bottom surfaces 21A/22A.

As illustrated in Fig. 5, the third member 30 is a cylindrical member having a cylindrical main body portion 30A and an annular portion 30B. The main body portion 30A has a first bottom surface 31, a second bottom surface 32, and a cylindrical cavity 33 opened in the second bottom surface 32. The annular portion 30B is provided on the second bottom surface 32.

As illustrated in Fig. 6, the fourth member 40 is a cylindrical member having a first bottom surface 41, a second bottom surface 42, and a cylindrical cavity 43 opened in the second bottom surface 42.

As illustrated in Fig. 7(b), the hollow cylindrical member 20A is configured so that the cylindrical member 20A can be inserted into the cavity 13 of the first member 10.

In addition, as illustrated in Fig. 5, an outer diameter r_{B} of the annular portion 30B of the third member 30 is larger than an outer diameter r_{A} of the main body portion 30A of the third member 30.

As illustrated in Fig. 7(c), the main body portion 30A of the third member 30 is configured so that the main body portion 30A can be inserted into the hollow cylindrical member 20A.

In addition, as illustrated in Figs. 3 and 6, a radius r₄₀ of the cavity 43 of the fourth member 40 is larger than an outer shape r₁₀ of the first member 10.

In addition, as illustrated in Fig. 2, threads may be formed on an inner surface of the fourth member 40 and an outer surface of the first member, and the first member 10 and the fourth member 40 may have a structure of a screw cap to be engaged by the threads.

Note that the mixture of the monomers containing the hydrophilic monomer for producing the endoscope hood is injected into a gap S illustrated in Fig. 2.

According to the configuration described above, since the hollow cylindrical member 20A can be separated into the two second members 20, releasing of the endoscope hood after a copolymerization reaction can be facilitated.

In addition, according to the configuration described above, since the first member 10 and the fourth member 40 have the structure of the screw cap, sealing property is improved, and it is possible to solve a problem that the mixture of the monomers containing the hydrophilic monomer for producing the endoscope hood leaks.

In addition, as illustrated in Figs. 2 and 5, the first bottom surface 31 of the third member 30 may be provided with a projection portion 35 for forming a forceps port serving as an inlet and an outlet of forceps and a projection portion 36 for forming a water supply port serving as an outlet of cleaning water in the obtained endoscope hood.

In the present embodiment, the endoscope hood is made of a hydrogel. Examples of the hydrogel include a hydrogel formed using only a hydrophilic monomer, and a hydrogel formed by adding a hydrophobic monomer, a crosslinkable monomer, or both to a hydrophilic monomer.

The hydrophilic monomer contributes to water content of the hydrogel, while the hydrophobic monomer contributes to an action of adjusting the water content and a swelling rate of the hydrogel, and affects wettability and flexibility of the obtained endoscope hood.

In addition, the crosslinkable monomer can control density of polymer chains of the hydrogel depending on a content thereof, and can impart mechanical strength, shape stability, and solvent resistance to the hydrogel.

As the hydrophilic monomer, those having one or more hydrophilic groups in a molecule are preferable, and examples thereof include 2-hydroxyethyl (meth)acrylate, 2-hydroxymethyl (meth)acrylate, hydroxypropyl (meth)acrylate, glycerol (meth)acrylate, acrylamide, N,N-dimethyl (meth)acrylamide, N,N-diethyl (meth)acrylamide, N-vinylpyrrolidone, diacetone acrylamide, N-vinylacetamide, (meth)acrylic acid, (meth)acryloxyethyl succinic acid, itaconic acid, methacrylamidopropyl triammonium chloride, 2,3-dihydroxypropyl (meth)acrylate, and the like; and two or more hydrophilic monomers may be used in combination among these.

A blending ratio of the hydrophilic monomer is not particularly limited, but is preferably 50 wt% or more based on all polymerized components because it affects the water content of the endoscope hood to be obtained. When the blending ratio of the hydrophilic monomer is less than 50 wt%, the endoscope hood having a sufficient water content cannot be obtained, which is not preferable since antifouling and antifogging properties of the endoscope hood may be deteriorated.

Examples of the hydrophobic monomer include siloxanyl (meth)acrylate, trifluoroethyl (meth)acrylate, methacrylamide, cyclohexyl (meth)acrylate, and normal butyl (meth)acrylate, and two or more of these hydrophobic monomers may be used in combination.

The hydrophobic monomer can change the water content of the endoscope hood obtained according to a blending amount. However, when a blending ratio of the hydrophobic monomer is high, the water content is extremely lowered, and the flexibility of the endoscope hood to be obtained is lowered. Therefore, for example, the blending ratio of the hydrophobic monomer is preferably less than 30 wt% with respect to a total amount of monomers.

Examples of the crosslinkable monomer include ethylene glycol di(meth)acrylate, methylene bisacrylamide, 2-hydroxy-1,3-dimethacryloxypropane, and trimethylolpropane triacrylate, and two or more of these may be used in combination.

A blending amount of the crosslinkable monomer is preferably 0.1 to 10 wt% with respect to the total amount of monomers from a viewpoint of an effect of adjusting the shape of the endoscope hood to be obtained. When the blending amount is less than 0.1 wt%, a mesh structure of the endoscope hood is insufficient, and when the blending amount exceeds 10 wt%, the mesh structure is conversely excessive, the endoscope hood becomes brittle and has decreased flexibility.

Examples of a polymerization initiator to be used for polymerizing a mixture of the monomers described above include peroxides such as lauroyl peroxide, cumene hydroperoxide and benzoyl peroxide which are general radical polymerization initiators; azobisvaleronitrile; and azobisisobutyronitrile. An addition amount of the polymerization initiator is preferably about 10 to 3500 ppm with respect to the total amount of monomers.

The endoscope hood according to the present embodiment is formed into a desired shape after a single or a plurality of the monomers described above are mixed, and is made of a single material.

As a step of obtaining a polymer, a monomer mixed solution obtained by mixing monomers as components is placed in the molding die of such as a metal, glass, or plastic and sealed, and a temperature is raised stepwise or continuously in a thermostatic bath or the like in a range of 30°C to 100°C. The copolymerization reaction is completed in 5 to 50 hours, whereby a molding die containing a polymer can be obtained. For polymerization, ultraviolet rays, electron beams, gamma rays, and the like can be used.

As a step of obtaining the hydrogel, the molding die after the polymerization is completed is cooled to room temperature, the polymer contained in the molding die is peeled off from the molding die, and the polymer is hydrated and swollen to form the hydrogel. Examples of the liquid (swelling liquid) to be used include, but are not limited to, water, physiological saline, isotonic buffer, and a solution obtained by mixing an organic solvent such as ethanol with these. The swelling liquid is heated to 60°C to 100°C, and the polymer is immersed in the swelling liquid for a certain period of time to be brought into a swollen state. In addition, it is preferable to remove unreacted monomers adhering to the polymer during the swelling treatment. The obtained hydrogel can be shaped by being subjected to high-pressure steam sterilization at 110°C to 130°C for 10 to 60 minutes while being immersed in the swelling liquid such as physiological saline.

Hereinafter, an example of a method for manufacturing the endoscope hood according to the present embodiment will be described with reference to Figs. 7(a) to 7(d).

First, as illustrated in Fig. 7(a), the two semi-cylindrical second members 20 are coupled to form the hollow cylindrical member 20A opened at both the bottom surfaces 21A/22A.

Second, as illustrated in Fig. 7(b), the hollow cylindrical member 20A is inserted into the cavity 13 of the first member 10.

Third, a mixture of monomers containing a hydrophilic monomer is injected into an opening of the hollow cylindrical member 20A.

Fourth, as illustrated in Fig. 7(c), the main body portion 30A of the third member 30 is inserted into the opening of the hollow cylindrical member 20A.

Fifth, as illustrated in Fig. 7(d), after an opening of the fourth member 40 is engaged with an outer periphery of the first member 10, heat treatment is performed in a range of 30°C to 100°C over 5 to 50 hours to obtain a copolymer. For example, the heat treatment is treatment of performing a copolymerization reaction by raising a temperature.

Sixth, after the first member 10 and the fourth member 40 are separated, the copolymer formed in voids thereof is taken out together with the second member 20A and the third member 30 from the first member 10, and then the second member 20A is divided.

Seventh, the copolymer adhering to the main body portion 30A of the third member 30 is immersed in a solution heated to 0°C to 100°C together with the third member 30 to be hydrated and swollen, thereby peeling off the hydrogel from the third member 30.

Eighth, the obtained hydrogel is immersed in the swelling liquid and shaped by being subjected to high-pressure steam sterilization at 110°C to 130°C for 10 to 60 minutes.

According to the molding die 1 for an endoscope hood according to the present embodiment, it is possible to facilitate releasing of the endoscope hood after the copolymerization reaction and to avoid liquid leakage during the copolymerization reaction.

As described above, the present invention has been described with reference to the embodiment described above, but it should be understood that the present invention is not limited to the description and drawings constituting a part of the disclosure in such an embodiment. Various alternative embodiments, examples, and operations will be apparent to those skilled in the art from such disclosure.

### REFERENCE SIGNS LIST

- 1: molding die for endoscope hood
- 10: first member
- 20: second member
- 20A: hollow cylindrical member
- 30: third member
- 30A: main body portion
- 30B: annular portion
- 35, 36: projection portion
- 40: fourth member
- 11, 31, 41: first bottom surface
- 12, 32, 42: second bottom surface
- 13, 33, 43: cavity
- r_{A}: outer diameter of main body portion
- r_{B}: outer diameter of annular portion
- r₁₀: outer shape of first member
- r₄₀: radius of cavity of fourth member

## Claims

1. A molding die for an endoscope hood comprising:
a cylindrical first member that has a first bottom surface, a second bottom surface, and a cylindrical cavity opened in the second bottom surface;
two semi-cylindrical second members that are coupled to each other to form a hollow cylindrical member;
a third member that includes a cylindrical main body portion that has a first bottom surface, a second bottom surface, and a cylindrical cavity opened in the second bottom surface, and an annular portion provided on the second bottom surface; and
a cylindrical fourth member that has a first bottom surface, a second bottom surface, and a cylindrical cavity opened in the second bottom surface, wherein
the hollow cylindrical member is open at both bottom surfaces,
the hollow cylindrical member is configured such that it is possible to inset the hollow cylindrical member into the cavity of the first member,
an outer diameter of the annular portion is larger than an outer diameter of the main body portion,
the main body portion is configured such that it is possible to insert the main body portion into the hollow cylindrical member,
a radius of the cavity of the fourth member is larger than an outer shape of the first member, and
the first member and the fourth member are engageable with each other.

2. The molding die for an endoscope hood according to claim 1, wherein
a projection portion is provided on the first bottom surface of the third member.

3. A method for manufacturing an endoscope hood, in which
a cylindrical first member has a first bottom surface, a second bottom surface, and a cylindrical cavity opened in the second bottom surface,
a third member includes a cylindrical main body portion that has a first bottom surface, a second bottom surface, and a cylindrical cavity opened in the second bottom surface, and an annular portion that is provided on the second bottom surface and has an outer radius larger than an outer shape of the main body portion, and
a cylindrical fourth member has a first bottom surface, a second bottom surface, and a cylindrical cavity opened in the second bottom surface,
the method comprising steps of:
coupling two semi-cylindrical second members to form a hollow cylindrical member opened at both bottom surfaces;
inserting the hollow cylindrical member into the cavity of the first member;
injecting a mixture of monomers containing a hydrophilic monomer into an opening of the hollow cylindrical member;
inserting the main body portion of the third member into the opening of the hollow cylindrical member; and
engaging an opening of the fourth member with an outer periphery of the first member to perform heat treatment.
